# EUROPEAN PATENT APPLICATION

(11) **EP 1 362 798 A1**
(43) Date of publication of application: **19.11.2003**
(21) Application number: 03252976.0
(22) Date of filing: 13.05.2003
(51) Int. Cl.: B65D 77/00, B65D 5/42

(54) **Double package**

(30) Priority: 14.05.2002 JP 2002139329
(71) Applicant: UNI-CHARM CORPORATION, Kawanoe-shi Ehime-ken (JP)
(72) Inventor: Morita, Yukiko, c/o Technical Center, Minato-ku Tokyo 108-8575 (JP); Ishikawa, Kentaro, c/o Technical Center, Minato-ku Tokyo 108-8575 (JP); Koguchi, Toshiyuki, c/o Technical Center, Minato-ku Tokyo 108-8575 (JP)
(74) Representative: Parry, Christopher Stephen

(57) **Abstract**

The invention provides a package system which is hard for others to notice when a user uses the product, but is superior in advertisement. The package system comprise a first package (2) in which the commodity product is contained, and a second package (1) in which a plurality of first packages (2) are contained. A trade name (5) may be provided on the first package (2) in such a size that it is not recognizable from a distant location. A design (4), which may be recognizable from the distant location, is provided on back and/or front surfaces of the second package (1).

## Description

### BACKGROUND OF THE INVENTION

### FIELD OF THE INVENTION

The invention relates to a package for exhibiting and selling a product, which is used preferably without being known to others. That is, a user would like to use the product, but the user would rather not show the user's usage of the product to others. The invention more specifically relates to a package to be used for containing a sanitary napkin, an interlabial pad, a tampon, a liner for menstrual exudate, an incontinence product, and a pair of sanitary shorts, or the like.

### BACKGROUND ART

A plurality of sanitary products of these types have hitherto been commonly exhibited for sale in the form of a single package. In order to make a trade name discernible from a distant location, the trade name is printed in large letters on front and back surfaces of the package. Further, with a view toward improving the convenience of a user who would otherwise have to purchase a plurality of packages, a second package into which the plurality of packages are packed is also offered for sale. A design is usually printed across the entire surface of the second package.

A user purchases a sanitary product or the like in the form of the package. When the user uses a sanitary product, the user takes a single product out of the package. Even if the user desires to keep usage of the product secret or at least not have the usage known to others, fulfilling the user's desire is impracticable so long as the product is taken out of the package, which has the trade name on the surface. The objective of keeping usage of the product secret may be attained if the trade name printed on the package is made small so as not to become recognizable from a distant location. However, the products cannot be recognized from such a distant location when exhibited for sale and hence become inferior in advertisement effect. In the case of the second package into which a plurality of packages are packed, a design is printed across the entire surface of the package. Hence, when the second package is exhibited, difficulty is encountered in ascertaining whether the single type of products are packed or a mixture of a plurality of types of products are packed.

### SUMMARY OF THE INVENTION

According to the present invention, it is an object to provide a package system characterized in that the product is hard to be recognized by others when the user open a single package containing the product and that the product is easy to be recognized by a customer or the user at the store when the user shops for the product.

According to the present invention, the package system comprises a first package for containing a commodity product and a second package containing a plurality of first packages. Here, containing may include packing and wrapping.

More specifically, a trade name, which may include a brand name, is provided on the first package packing a commodity product in such a size that the name is not recognizable from a distant location. A plurality of first packages are packed, thereby forming a second package. The trade name is provided on the front and back surfaces of the second package in such a size that the name becomes recognizable from the distant location. When the second package is placed on a display shelf for sale, the trade name of the second package can be perceived from a distant location. However, when the commodity product is picked from the package for use, the trade name provided on the first package could be more difficult to perceive. In other words, it is not easy for others to recognize the trade name provided on front and/or back surfaces of the first package containing the product.

More specifically, the following is provided according to the present invention.

(1) A double package containing a commodity product which is used preferably without being known to others comprises a first package in which the commodity product is contained and a second package in which a plurality of first packages are contained; wherein a trade name is provided on front and/or back surfaces of the first packages in such a size that the trade name is not recognizable from a distant location; and wherein a design is provided on front and/or back surfaces of the second package in such a manner that the design is recognizable from the distant location.

There is provided a double package which packs a commodity product whose use is desired to be maintained secret and which is formed from a first package into which the commodity product is packed, and a second package into which a plurality of first packages are wrapped, wherein a trade name is provided on back and/or front surfaces of the first package in such a size that the trade name is not recognizable from a distant location, and a design which is recognizable from a distant location is provided on back and/or front surfaces of the second package.

According to the present invention, the second package may contain a plurality of first packages. Each first package may have a trade name provided on front and/or back outer surfaces of the first package in such a size that the name is not noticeable from a distant location. The second package may have a design provided on front and/or back outer surfaces of the second package in such a size that the design is noticeable from a distant location. Therefore, the commodity product may be noticeable from the distant location since it is packed in the second package and is placed on a display shelf for sale. Here, a noticeable design or trade name is recognizable. However, when a user takes the commodity product out of the first package, the trade name, which is not noticeable, provided on the front and/or back surfaces of the first package may be hard to perceive from the distant location, thereby letting other people not know what the commodity product really is. Specifically, when the commodity products are placed on the display shelf for sale, the package system renders identification and advertisement as well as having intimacy and secrecy of the commodity product secret when it is used. Here, the design and the trade name may be provided on either the front or back surface of the package or on both surfaces of the same.

Here, the term "first package" may mean a package packing a commodity product. The first package is not limited to any particular packaging form; that is , the first package may include a package being made from or of any materials and formed into any shapes. The front and back surfaces of the package may comprise a face or surface which faces customers when the commodity products are placed on the display shelf: Hence, the front or back surface may be provided with a trade name, a design, and the like, to appeal to the customers.

(2) In the double package according to (1), the second package comprises a wrapping package which wraps outer peripheral surfaces of the plurality of first packages with a transparent or translucent film. Preferably, the second package is formed by wrapping outer peripheral surfaces of the plurality of first packages with a transparent or partially transparent film.

According to the present invention, the first packages may be wrapped with a transparent or partially transparent film, thereby constituting the second package. Therefore, even when the commodity products are packed double, the user can purchase commodity products with the confidence that the product is what the user wants. Since indications provided on the exterior surface of the first package can be seen through the transparent or partially transparent film, only an indication desired to be perceived or notices from the distant location may be provided on the exterior surface of the second package. Therefore, the exterior surface of the second package may not have to have detailed descriptions such as precautions since the user can read such indications on the first packages through the film when the user picks up the commodity product being packed in the second package. Therefore, printing the same indications on the filmmaybe omitted. Therefore, printing and other costs can be curtailed. Here, it is preferable to use a plastic film such as a polyethylene film, a polypropylene film, and so on, which is generally used as a wrapping film and comprises a transparent or translucent film so that the user can visually ascertain the indication provided on the first package. Particularly, a transparent film is more preferable since it is easy to ascertain the contents.

(3) In the double package according to (1), the second package contains the plurality of first packages which contain a same kid of commodity product. Preferably, the second package packs the plurality of first packages respectively formed from the same commodity products.

According to the present invention, the second package is made by packing a plurality of first packages. It is convenient for the user to collectively purchase a plurality of commodity products and for the seller to place the products on the display shelf. Packaging a plurality of same commodity products may include packing a plurality of single products and packing a plurality of combined products (e.g., a combination of scented liners for menstrual exudate and unscented liners for menstrual exudate).

(4) In the double package according to (1), the design may be drawn within a front or back area of one of the first packages, and the second package may comprise a transparent surface part within the front or back area of the one of the first packages. Further, in the double package according to (1), the design may be drawn within a front or back area of one of the first packages, and the second package may comprise a transparent surface part within a second front or back area of another of the first package. Yet further, in the double package according to (1), the design may be drawn over a front or back area of one of the first packages, and the second package may comprise a transparent surface part within the front or back area of the one of the first packages, and the design may comprise a continuous design part over the front or back area of the one of the first packages. Preferably, a design covering one piece of the first package is provided on front and/or back surfaces of the second package and in substantially the same size as that of the first package with a transparent portion being provided, and a portion of the design is provided so as to continuously extend to a remaining portion of the first package.

According to the present invention, the design may be provided on the front and/or back surfaces of the second package substantially in the same size as one piece of the first package. However, a combination of a transparent portion and a designed portion, which may not be transparent or translucent either, may cover the same size of the front or back surface of one of the first package. The design may have a continuously extending portion beyond the designed portion corresponding to the same size of the front or back surface of the one first package. The design may be substantially in the same size as the one first package or smaller than the size of the one first package. The design may be made in a size and shape independently from that of the first package.

The second package may comprise a plurality of first packages as mentioned above and the first packages may be arranged in a flat form in the second package. For example, the first packages may be put and aligned on a plane side-by-side with back surfaces of the first packages placed on the plane. Therefore, the front or back surfaces of the second package may have a plurality of sections or parts corresponding to the front or back surfaces of the first packages. Since the second package may comprise a film covering such parts continuously, the design may be consistently drawn on the film beyond each part with ease. Therefore, the design may be much bigger than the front or back surface of the one first package while the part corresponding the surface of the one first package may have a transparent or translucent portion within the area of the part such that the user may see some of the first packages through the transparent or translucent portion. Hence, the package exhibits an image of the first package being a single set. The design is usually provided on the film member by means of printing. However, it is not limited to printing. For instance, a label having a design printed thereon is affixed to the surface of a film member. Alternatively, a design is printed on paper or the like, which is placed between the first and second packages such that the design may be seen through the transparent film member. The design printed on the second package may be so translucent that indications, such as a design and a trade name, provided on the first packages may be seen over the design.

According to the present invention, the term "front and back surfaces of the package" may mean surfaces which face customers when the commodity products are placed on the display shelf. Such surfaces may bear the trade name and the design provided for appealing customers. Here, the design may include a picture, a figure, an illustration, a sketch, an ornamental pattern, a mark, a sign, a logo, an indicia and so on. The design may include a figure, a character including a foreign character, an alphabet, and a combination thereof. The design may further include a trade name, the type of a commodity product, a corporate name, a catch phrase, and so on. Here, the design may comprise such element mentioned above or a combination thereof.

(5) In the double package according to (1), the design may comprise an associating design in association with a product image of the commodity product. Preferably, a trade name which is recognizable from the distant location may be provided on the second package and at substantially a center position on a part corresponding to a front and/or back surface of the first package.

According to the present invention, a trade name may be provided at each position on the front and/or back surfaces of the second package corresponding to each center position on respective packed first packages in such a size that the trade name can be recognized from the distant location. Therefore, the trade name can be readily identified from the distant location. Further, the trade name is provided at positions on the upper surface of the second package corresponding to the respective pieces of the first package. Specifically, the trade name is printed as many times as the number of the first packages contained in the second package. Hence, in cooperation with the indications provided on the area extending continuously to the design provided on the front and back surfaces, the second package yields the effect of enabling the user to readily ascertain the number of the packed first packages.

(6) The second package according to any of (1) through (5) is characterized in that a plurality of wrapped first packages inside can be visibly perceived through a transparent portion.

According to the present invention, the second package is made by wrapping the first package with a transparent or partially transparent film member. The design, including a trade name which can be perceived from the distant location, may be provided on the front and/or back surfaces of the second package. Since the indication of the design does not extend across the entire exterior surface of the second package, if the user wishes, the user can readily view details provided on the first package.

(7) In the package according to any one from (1) to (5), the design may inherits a display form on which an image of commodity product has been built up over the years.

According to the present invention, the design to be provided on the front and/or back surfaces of the second package inherits the design for commodity products, the design being a successor of a conventional display form into which the image of commodity products has been accumulated over the years. Hence, even when the design is viewed from the distant location, the user can readily recognize the nature of the commodity products while glancing at only the design. Accordingly, when the user purchases commodity products, the user can readily find desired commodity products and pick up the correct commodity products without ascertaining details and without involvement of misidentification. Further, when commodity products are offered for sale, they can be perceived without large-scale advertisement, because they have already been known. When the design is drastically changed in the future, the design will act as a bridge between the old design and a new design. Specifically, the design which is about to be subjected to drastic changes is provided on pieces of the first package packed in the second package. As a result, a significantly-changed design can be fixed as an image design for the commodity products by displaying it on the first package packed in the second package while the commodity products are sold together with the traditional image design of the commodity products.

(8) The double package according to (1), wherein the trade name is provided at substantially a center position on the front and/or back surfaces of the first package. The package according to any one from (1) to (6) is characterized in that the trade name of the commodity product contained in the first package is provided at substantially a center position on front and/or back surfaces of the first package and in a such a size that the trade name cannot be perceived from the distant location.

According to the present invention, the design, for example, a trade name, provided on the front and/or back surfaces of the first package, is provided at substantially the center positions in such a size that the trade name cannot be recognized from the distant location. Hence, when the user uses a commodity product by picking it up from the first package, the nature of the product can be maintained secret from other persons.

(9) The double package according to (8) may be characterized in that each design corresponding to each kind of the commodity product is provided on the front and/or back surfaces of the first package. Preferably, a design which changes from one commodity product to be packed to another is provided on the front and/or back surfaces of the first package.

According to the present invention, the design provided on the front and/or back surfaces of the first package changes according to the type of a commodity product packed in the first package. The second package wraps the first package with a film member such that a transparent portion is provided. Hence, the user can view contents of the first package. Therefore, visual ascertainment of the design on the first package eliminates the risk of the user's erroneously purchasing unwanted commodity products, because the user can ascertain the type of the commodity product.

(10) The double package according to (9) may be characterized in that a description of the commodity product is provided on a lateral surface of the first package. Preferably, a description of the packed commodity product and a sales tool are provided on lateral surfaces of the first package.

According to the present invention, descriptions and characteristics of packed commodity products and a sales tool are provided on lateral surfaces of the first package. The second package wraps the first package with a film member such that a transparent portion is provided, thereby enabling the user to view contents of the first package. Consequently, the indications printed on the first package can be seen through the second package and visually checked by way of the second package. Further, there is no necessity for causing the indications to be perceived from the distant location. Hence, enlarged provision of the indications is not necessary. For these reasons, repeated printing of the same indications on the second package is not required, and hence printing costs and other costs can be curtailed.

### BRIEF DESCRIPTION OF THE DRAWINGS

Fig. 1A shows a perspective view of a first embodiment in a Japanese version according to the invention.
Fig. 1B shows a perspective view of a first embodiment in an English version according to the invention.
Fig. 2A shows a perspective view of a first package in a Japanese version.
Fig. 2B shows a perspective view of a first package in an English version..
Fig. 3A shows a perspective view of a second embodiment in a Japanese version.
Fig. 3B shows a perspective view of a second embodiment in an English version.

### DETAILED DESCRIPTION OF THE PREFERRED EMBODIMENTS

Embodiments of the invention will be described hereinbelow by reference to the drawings.

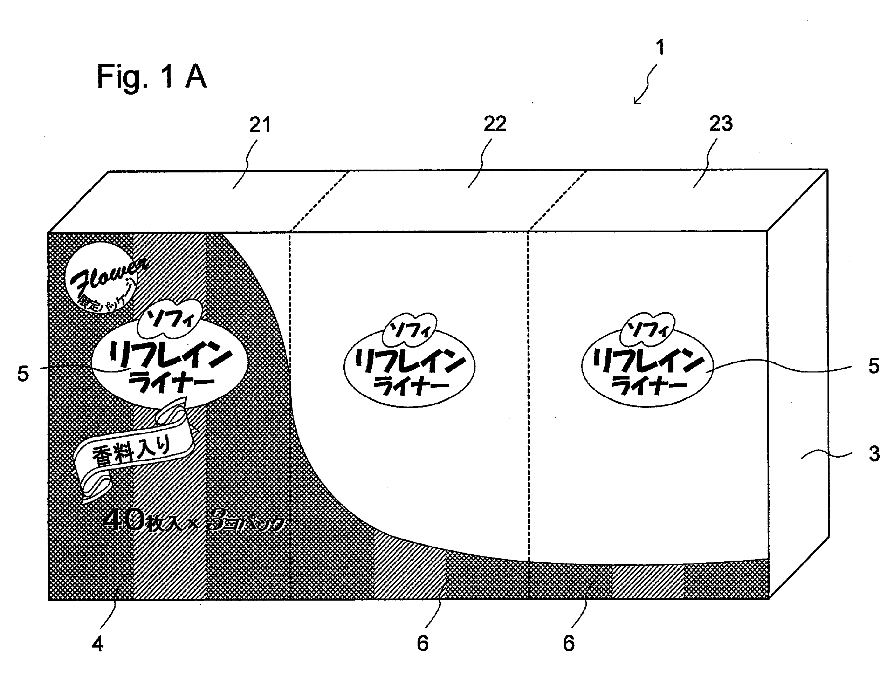

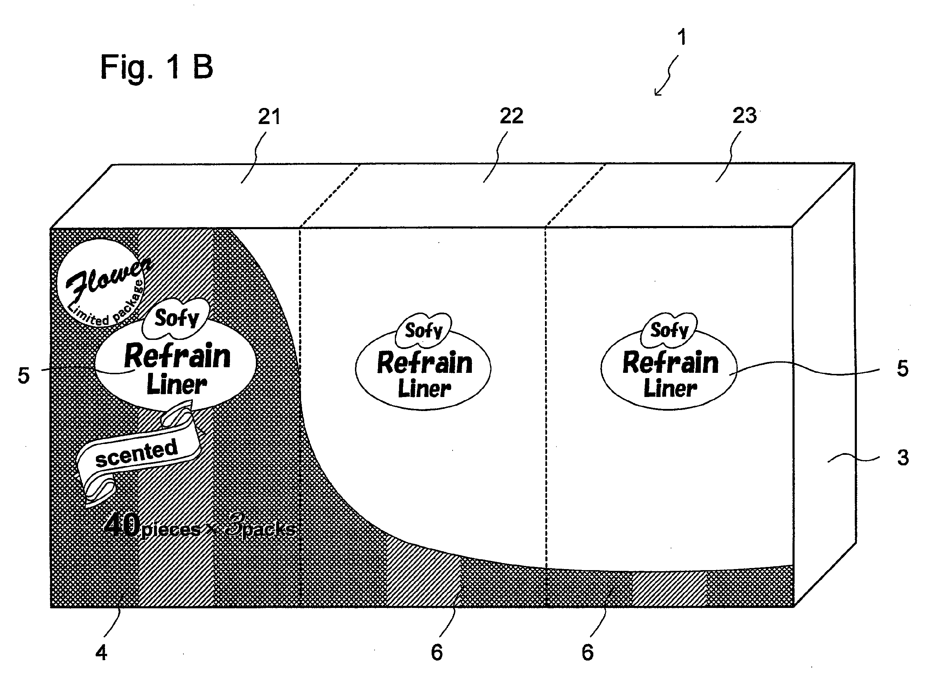

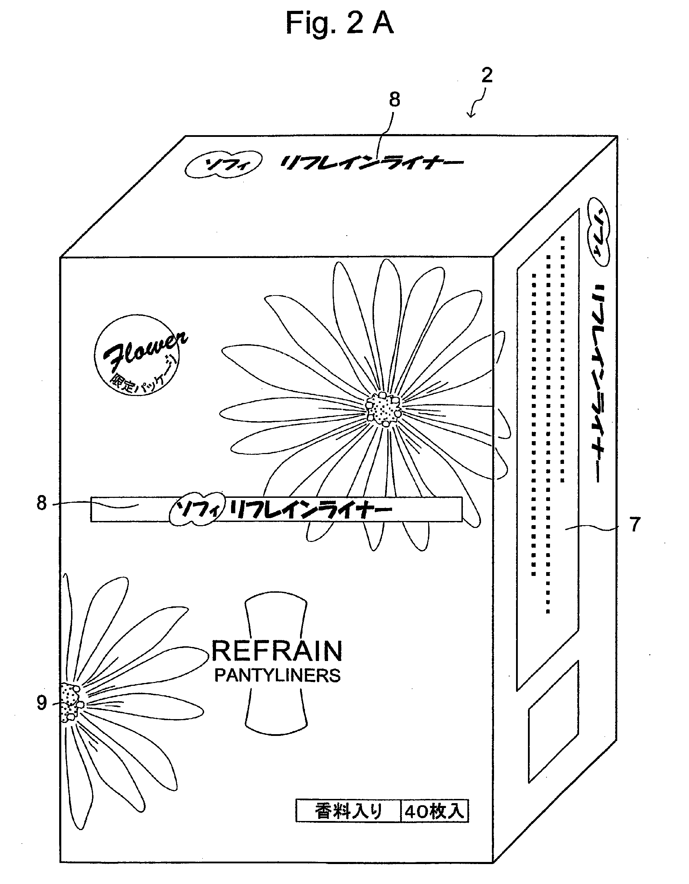

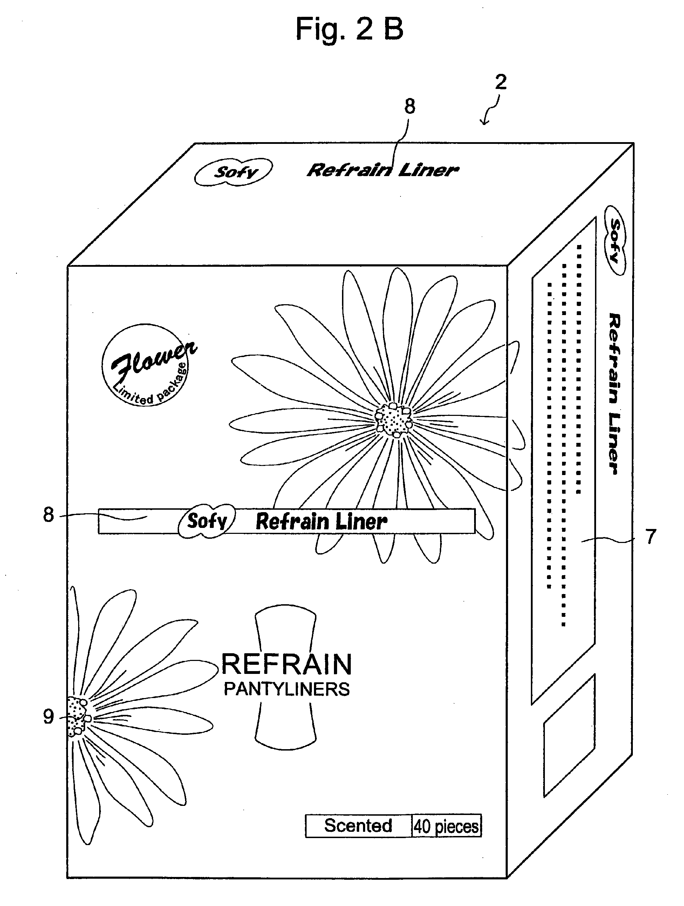

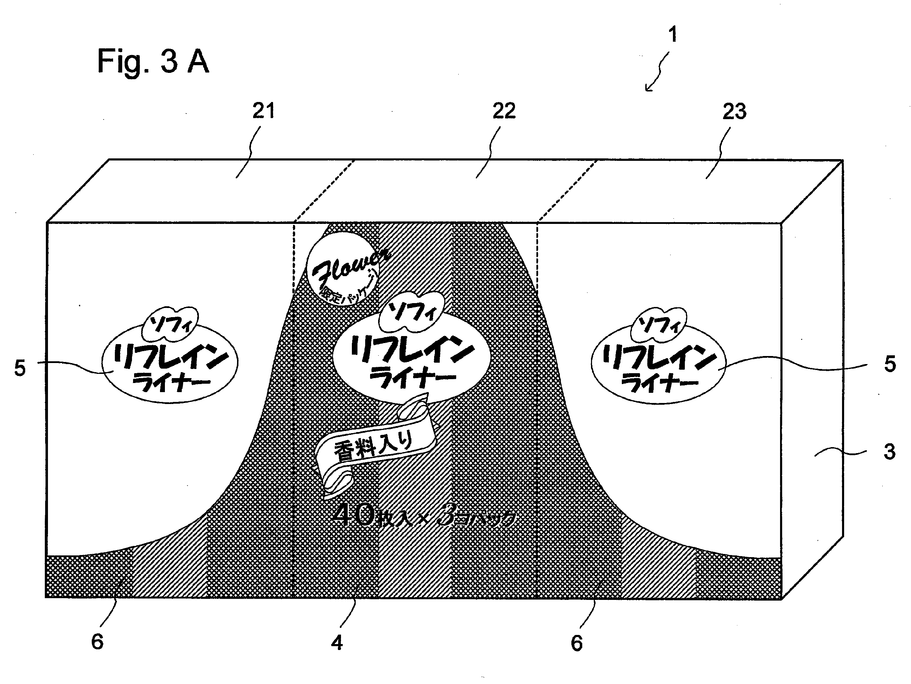

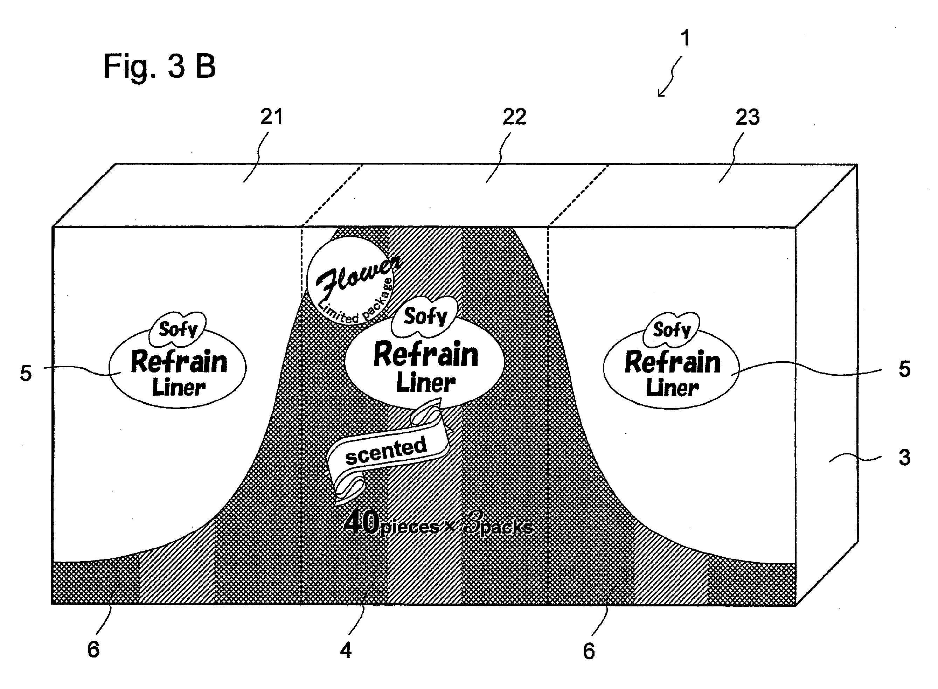

Fig. 1A or 1B shows a perspective view of a first embodiment of a package in a Japanese or English version according to the present invention. Fig. 2A or 2B shows a perspective view of a first package in a Japanese or English version according to the present invention. Fig. 3A or 3B is a perspective view of a second embodiment in a Japanese or English version according to the present invention.

### [First Embodiment]

As shown in Fig. 1A or 1 B, the second package 1 is formed by collectively shrink-wrapping three first packages 21, 22, and 23, each of which has the same shape as shown in Fig. 2A or 2B with the numerical reference of 2, with a film member 3. Specifically, the first package 2 is formed into the shape of a long rectangular parallelepiped by collectively packing a plurality of commodity products. The film member 3 is a transparent or partially transparent or translucent film. The film member 3 may be embodied by a commonly-available single film made of polyethylene or polypropylene or by a commonly-available laminated filmmade by laminating single films. A ribbon-shaped tape of small width (not shown) is provided at an upper location on the surface or an upper side surface of the second package 1 for unwrapping the package.

A trade name 5 with a simple illustration, a product type, and a design 4 including the number of contents and the number of combinations are printed for display on the front and back surfaces of the second package 1 in a size substantially identical to that of the first package piece 21 located at the leftmost position. The figure shows the front surface of the second package. A part 6 of the design 4 is extending continuously in rightward across the portions corresponding to the first packages 22, 23 of the second package. The design 4 is printed to cover most area of a part, which corresponds to the front surface of the first package 21, of the front surface of the second package 1. In an upper right portion of the part corresponding to the front surface of the first package 21, the design 4 is not printed, but the portion is left transparent so as to enable the user to view the contents inside the second package. The portion 6, which is considered to be a part of the design 4, is printed on areas corresponding to the first packages 22, 23 so as to continuously extend to the lower end portions of the second package. The design 4 is drawn in such a size that information such as a trade name, a figure, the type of a commodity product (the name of a type) , and a volume of contents, can be perceived from the distant location. The design 4 may inherit a display form, onto which an image of the commodity product has been accumulated over the years, and may be modified during such history. The design 4 enables buyers (including the user) to readily perceive the product at a glance, thereby advertising the commodity product. The design 4 may inherit the image of the commodity product, which has been accumulated over the years by using the design. When a new design is made by changing the original old design drastically, the design 4 may serve as a bridge between the old design and the new design. For example, a drastically-changed new design may be printed on the first package and the bridging design 4 may be printed on the second package so that the buyer may purchase the right product because of the design 4 and the buyer or the user will be familiar with the drastically-changed design by using the product taken out of the first package which has the drastically-changed new design.

The trade name 5 may be printed substantially at the center of each part corresponding to each first package 21, 22, 23 substantially in the same size as is recognized from the distant location. The number of the printed trade names 5 is equal to the number of the first packages 21, 22, 23 contained in the second package 1. As a result, the user can perceive the number of the first packages contained in the second package 1 from the distant location.

The design 4 and the trade name 5 are printed on the surface of the film member, but it is not limited to the film member. The design 4 and the trade name 5 may be provided on the package by means of affixing a label on the surface of the package. Printing does not necessarily involve use of any special ink or printing method but may use a generally-employed material and method. The portion 6 of the design 4 is printed on the areas corresponding to the remaining first package pieces 22, 23 and so as to continuously extend to the lower end portions of the parts corresponding to the first package pieces 22, 23, but it is not limited to the lower end portions. The portion 6 may be printed at upper end portions of the parts corresponding to the first package pieces 22, 23.

In this way, the design 4 may not have to be printed across the front and back surfaces of the second package. Hence, the user can visually ascertain an indication of the first package 2 (in Fig. 2) of the commodity product by way of a transparent or partially transparent portion of the second package where no design is printed to leave it blank. Accordingly, there can be obviated a risk of the user erroneously purchasing unwanted commodity products. The portion 6 of the figure of the design 4 extends over the remaining first package pieces 22, 23 so that the first packages 21, 22, 23 wrapped with the second package 1 may be considered as a single product because of the single design 4.

Figs. 1A and 1B show Japanese and English versions of the second packages 1. The trade name 5 and other information written in Japanese or English language appeal directly to the people who speak Japanese or English, or both. Therefore, it is another way to use characters of more than one languages since a familiar language is easy to be recognized while an unfamiliar language does not make sense to people who cannot understand the language. Thus, the user may take the product out of the first package 2 (in Figs. 2A and 2B) without being notified by others who do not understand the language even though the trade name is written in such a size that people who understand the language can recognize it from the distant location. The analogy may apply to the following explanation and the drawings in Figs. 1A, 1B, 2A, 2B, 3A, and 3B.

Lateral surfaces and the top and bottom surfaces of the second package 1 remain unprinted. These areas are transparent or partially transparent, thereby enabling the user to view the indication of the first package 2 packed in the second package 1. As a result, the user can view descriptions provided on the lateral surfaces of the first package 2 (in Figs. 2A and 2B). Indication 7 of information required by the law or other reasons is provided on the lateral surface of the first package 2 and descriptions and characteristics of the commodity product, a sales tool, and the like can be seen through the second package so as to achieve the objective even when the information indication 7 is not provided on the second package 1. Thus, the user can ascertain the information indication when the user purchases the commodity product. For this reason, since repeated printing of the same indications on the second package 1 is not required, printing expenses or the like can be curtailed. When a bar code, or the like, which indicates a price, is printed on the first package 2, but is not shown in the drawing, a symbol is preferably printed at locations corresponding to the bar code for concealing the bar code, thereby disabling reading of the bar code provided on the first package 2.

So far the embodiment, in which the design 4 is printed on the front surface of the first package 21 located at the leftmost end in the second package 1, has been described. The design 4 may be printed on the front surface of the first package piece located at the rightmost end in the second package. Further, the design 4 may be printed on either the front or back surface of the first package or on respective surfaces of the same. When the design is printed on the front and back surfaces, the designs provided on the front and back surfaces may preferably be the same. By means of this arrangement, the same designs face customers even when commodity products are arranged on the display shelf, such that the customers do not have to check the front surfaces thereof. Thus, printing a single design on both surfaces of the package is more desirable, in view of achievement of good appearance and elimination of labor which would otherwise be required when commodity products are arranged on the display shelf.

The second package 1 of the embodiment is made by wrapping the plurality of first package pieces 21 to 23 with the transparent film member 3 and by printing the design 4 on the film member 3. However, the invention is not limited to the film member 3. For instance, the design may be printed on, e.g., paper. The first package 2 may be wrapped in conjunction with the paper corresponding to the printed design with a transparent filmmember. Thus, another medium for showing a design may be sandwiched between the first and second packages.

The first package 2 is described. The first package 2 is a substantially-rectangular parallelepiped, in which a plurality of commodity products are packed. As shown in Fig. 2, the first package 2 is made by wrapping a plurality of commodity products with a filmmember such as polyethylene or polypropylene. The design 4 including a flower design 9 is printed at a lower left position and an upper right position on each of the front and back surfaces of the first package 2. Further, a trade name 8, which is not recognizable from the distant location, is printed substantially at the center of each front surface and each back surface. Thus, as a result of adoption of the flower design 9, which is totally different from the design of the traditional image of the commodity product that has been built up over the years , it is suggested that this is a special product with limited package and the product may be purchased by a user who renders a prejudice against the traditional image of commodity product for years. Here, the design is not limited to a flower design. The required information indication 7, such as descriptions of the commodity product and the sales tool, is printed on both lateral surfaces of the first package 2. The trade name is printed on upper and lower surfaces of the first package 2 in such a size that others cannot ascertain the trade name from the distant location. By means of the indication, the user purchases a commodity product while visually checking the performance of the commodity product. The packing form of the first package 2; that is, the material and shape of the first package 2, is not limited to those mentioned above. The first package 2 may be formed from a paper box, and the indications may be provided on the first package 2 not by printing but by means of a label or the like. Further, the ink and printing method employed for printing operation are not necessarily special but may be embodied by a commonly-available material and printing method.

### [Second Embodiment]

Figs. 3A and 3B show a perspective view of a package of a second embodiment in a Japanese version and an English version, respectively, according to the present invention. In the following embodiment, similar elements may be referred to by similar references numerals.

As in the case of the first embodiment, according to the second embodiment, the second package 1 is made by collectively shrink-wrapping the three first packages 21, 22, and 23 with the film member 3. Specifically, each first package 21, 22, 23 is formed into a rectangular parallelepiped by collectively packing a plurality of commodity products. The film member 3 is a transparent or partially transparent or translucent film. The film member 3 may be embodied by a commonly-available single film made of polyethylene or polypropylene or by a commonly-available laminated film made by laminating single films. A ribbon-shaped tape of small width (not shown) is provided at an upper location on the surface or an upper side surface of the second package 1 for unwrapping the package.

The design 4 is provided for display on the front and back surfaces of the second package 1 at the position corresponding to the center position of the first package 22 being located in the middle of the second package 1. The design 4 is printed to cover most area of a part, which corresponds to the front surface of the first package 22 of the front surface of the second package 1. Upper right and left portions of the part corresponding to the first package 22, where the design 4 is printed, are transparent so as to enable the user to view the content inside the second package 1. Portions 6, 6 of the design 4 are printed on lower end portions of the second package 1 corresponding to lower end portions of the first packages 21, 23, respectively, so that the design 4 continuously extends to both sides. Both lateral surfaces (or right and left surfaces) and top and bottom surfaces of the second package 1 remain transparent without any designs or trade names.

As in the case of the first embodiment, the design 4 may comprise information such as a trade name, a figure, a type of a commodity product (type name) , and a volume of contents, which are printed in such a size that they can be perceived from the distant location. The design 4 inherits a display form, onto which the image of the commodity product has been accumulated for years. The design 4 is modified from the display form. The design 4 enables buyers to readily perceive the product at a glance, whereby the commodity product is advertised. Since the design 4 inherits the display form onto which the image of the commodity product has been accumulated for years, the user can readily perceive the commodity product.

As in the case of the first embodiment, the trade name 5 is printed substantially at the center of each part correponding to each first package 21, 22, 23 substantially in the same size. The number of printed trade names 5 is equal to the number of first packages 21, 22, 23 wrapped in the second package 1. As a result, the user can perceive from the distant location the number of the first packages having commodity products packed in the package 1.

Although the design 4 and the trade name 5 are printed on the surface of the film member, they may be provided on the package by means of affixing a label on the surface of the package, thereby not being limited to the film member. Printing does not necessarily involve use of any special ink or printing method, but a generally-employed material and method may be employed.

In this way, since the design 4 is not printed across the front and back surfaces of the second package, the user can visually ascertain an indication of the first package 2 of the commodity product by way of a transparent or partially transparent portion of the second package where no design is printed and the portion is left blank. Accordingly, there can be obviated a risk of the user erroneously purchasing unwanted commodity products. The portion 6 of the figure of the design 4 extends over the remaining first package pieces 21, 23. The first package 21, 22, 23 wrapped with the second package 1 yields an effect of imparting an image of one product as far as the design 4 covers.

As in the case of the first embodiment, the design 4 may be printed on either the front or back surface of the first package 2 or on both surfaces. When the design is printed on the front and back surfaces, the designs provided on the front and back surfaces may preferably be the same. Consequently, the same designs face customers even when commodity products packed in the second package are put on the display shelf, without checking if the second package is put backward. Thus, printing one kind of design on both front and back surfaces of the package is more desirable, in view of achievement of good appearance and elimination of labor which would otherwise be required when commodity products are arranged on the display shelf. Further, the design may be printed on, e.g., a piece of paper. The first package may be wrapped with the piece of paper having the printed design and wrapped with a transparent film member instead that the first packages are wrapped with the film member 3 on which the design 4 is printed. Thus, another medium for showing the design may be placed between the first and second packages.

As in the case of the first embodiment, lateral surfaces and top and bottom surfaces of the second package 1 are left unprinted. These areas are transparent or partially transparent, thereby enabling the user to view the indication of the first package 2 packed in the second package 1. As a result, the user can view descriptions provided on the lateral surfaces of the first package 2. Therefore, even when the description of the commodity product and the sales tool, which are displayed on the lateral surfaces of the first package 2, may not be displayed on the second package 1 so that the user can ascertain these indications from the distant location, the buyer can view the indication when the buyer purchases the commodity product at a store. For this reason, since repeated printing of the same indications on the second package 1 is not required, printing expenses or the like can be curtailed.

In the embodiments described before, the design printed on the second package appears on the front and/or back surface of the second package substantially in the same size as a front surface of one first package so as to cover most front surface of the first package while transparent portions are left. The design provided on the front or back surface may extend continuously across front surface parts corresponding front surfaces of the first packages. A design may be printed over the entire front and back surfaces of the second package, and a portion on which the design is printed may be translucent so that the user can visually check the indication provided on the surface of the first package.

It should be understood that the package according to the present invention is not limited to the embodiments and may be made for various modifications without departing from the scope of the present invention.

For instance, although the second package is made by collectively packing three first packages, two, four, or more first packages may be packed. It is preferable to have the design on which the image of the commodity product is accumulated thus far, since the design enables the user to perceive the commodity product at a glance. However, it is not limited to such design.

As described before, the package system, according to the present invention, is composed of a first package in which the commodity product, which the user would rather not let others know the user use, and a second package in which a plurality of first packages are contained. The trade name is provided on front and back surfaces of the first package in such a size that the trade name is not recognizable from the distant location. The design which is recognizable from the distant location is provided on the back and front surfaces of the second package. Therefore, when the package is arranged on the display shelf for sale, the user can perceive the commodity product from the distant location. When the user uses the product, the trade names provided on the front and back surfaces of the first package are not recognizable from the distant location, thereby keeping it private that the user uses the product. Further, the invention may yield the effect of advertising the commodity product in a recognizable manner when the product is arranged on the display shelf for sale, as well as the effect of keeping it private that the user uses the product.

The designs provided on both front and back surfaces of the second package may inherit the display form that has been built up thus far by marketing the commodity product. Therefore, the design can be readily perceived from the distant location if it is arranged on the display shelf. Hence, the user can readily identify the design at the time of purchasing the product, obviating a chance of erroneously purchasing a wrong commodity product.

The second package is formed of a transparent film member serving as a package member. Only the trade name and the design are provided on the front and back surfaces of the second package in combination with transparent portions. Hence, the content of the second package can be seen through the second package, whereby the customer can read information such as descriptions of a commodity product provided on the first package. Therefore, the user can ascertain the commodity product the user wishes to purchase. Further, printing costs or the like can be curtailed.

## Claims

1. A double package containing a commodity product which is used preferably without being known to others, the double package comprising:
a first package in which the commodity product is contained and
a second package in which a plurality of first packages are contained;
wherein a trade name is provided on front and/or back surfaces of the first packages in such a size that the trade name is not recognizable from a distant location, and
wherein a design is provided on front and/or back surfaces of the second package in such a manner that the design is recognizable from the distant location.

2. The double package according to claim 1, wherein the second package comprises a wrapping package which wraps outer peripheral surfaces of the plurality of first packages with a transparent or translucent film.

3. The double package according to claim 1, wherein the second package contains the plurality of first packages which contain a same kid of commodity product.

4. The double package according to claim 2, wherein the second package contains the plurality of first packages which contain a same kid of commodity product.

5. The double package according to claim 1, wherein the design is drawn within a front or back area of one of the first packages, and
wherein the second package comprises a transparent surface part within the front or back area of the one of the first packages .

6. The double package according to claim 2, wherein the design is drawn within a front or back area of one of the first packages, and
wherein the second package comprises a transparent surface part within the front or back area of the one of the first package.

7. The double package according to claim 3, wherein the design is drawn within a front or back area of one of the first packages, and
wherein the second package comprises a transparent surface part within the front or back area of the one of the first package.

8. The double package according to claim 1, wherein the design is drawn within a front or back area of one of the first packages, and
wherein the second package comprises a transparent surface part within a second front or back area of another of the first package.

9. The double package according to claim 1, wherein the design is drawn over a front or back area of one of the first packages,
wherein the second package comprises a transparent surface part within the front or back area of the one of the first packages, and
wherein the design comprises a continuous design part over the front or back area of the one of the first packages.

10. The double package according to claim 1, wherein the design is drawn over a front or back area of one of the first packages,
wherein the second package comprises a transparent surface part within the front or back area of the one of the first packages, and
wherein the design comprises a continuous design part over the front or back area of another of the first packages.

11. The double package according to claim 1, wherein the design is drawn over a front or back area of one of the first packages,
wherein the second package comprises a transparent surface part over the front or back area of the one of the first packages, and
wherein the design comprises a continuous design part over the front or back area of the one of the first packages.

12. The double package according to claim 1, wherein another trade name being recognizable from the distant location is provided on the front and/or back surfaces of the second package and at a position corresponding to a substantially center position of the front and/or back surfaces of the first packages.

13. The double package according to claim 2, wherein the second package enables visible perception of the plurality of wrapped first packages.

14. The double package according to claim 1, wherein the design comprises an associating design in association with a product image of the commodity product.

15. The double package according to claim 1, wherein the trade name is provided at substantially a center position on the front and/or back surfaces of the first package.

16. The double package according to claim 15, wherein each design corresponding to each kind of the commodity product is provided on the front and/or back surfaces of the first package.

17. The double package according to claim 16, wherein a description of the commodity product is provided on a lateral surface of the first package.
